# EUROPEAN PATENT APPLICATION

(11) **EP 4 458 973 A1**
(43) Date of publication of application: **06.11.2024**
(21) Application number: 22911810.4
(22) Date of filing: 19.12.2022
(51) Int. Cl.: C12N 15/86, A61K 48/00, A61P 35/00, C07K 14/005

(54) **IMMUNE-CLOAKING ANTITUMOR ADENOVIRUS**

(30) Priority: 21.12.2021 KR 20210183522; 21.12.2021 KR 20210183523; 21.12.2021 KR 20210183524; 21.12.2021 WO PCT/KR2021/019511
(71) Applicant: Curigin Co.,Ltd., Seoul 04778 (KR)
(72) Inventor: LEE, Wan, Iksan-si Jeollabuk-do 54658 (KR); YOO, Jung Ki, Yangju-si Gyeonggi-do 11456 (KR); UM, Ki Hwan, Ansan-si Gyeonggi-do 15338 (KR); KIM, Mi Soon, Seoul 04796 (KR); CHOI, Jin Woo, Seongnam-si Gyeonggi-do 13559 (KR)
(74) Representative: Isarpatent
(86) International application number: PCT/KR2022/020712
(87) International publication number: WO 2023/121177

(57) **Abstract**

The present invention relates to an antitumor adenovirus capable of evading the in vivo immune system. According to the present invention, the adenovirus comprising a nucleic acid coding for a transferrin-binding domain of the present invention exhibits notably increased effects of infecting and killing tumor cells, exhibits increased binding to transferrin, thereby evading an in vivo immune response and thus increasing the plasma half-life, has a systemic therapeutic effect by being specifically delivered to cancer cells, is capable of being topically delivered, and has excellent selectivity, thereby exhibiting the effect of notable antitumor efficacy, and thus may be usefully employed as an anticancer composition or an anticancer adjuvant for various carcinomas.

## Description

### [Technical Field]

The present invention relates to an antitumor adenovirus capable of evading the in vivo immune system.

### [Background Art]

Cancer is one of diseases that cause the most deaths worldwide, and the development of innovative cancer therapy is able to reduce medical costs incurred during treatment of the cancer and create high added value at the same time. In addition, according to statistics from 2008, molecular therapeutic agents that may overcome resistance to existing anti-cancer drugs accounted for $17.5 billion in seven major countries (US, Japan, France, Germany, Italy, Spain, and UK), and in 2018, accounted for the market size of approximately $45 billion, which is expected to show a growth rate of 9.5% compared to 2008. Cancer therapy is divided into surgery, radiation therapy, chemotherapy, and biological therapy. Among them, the chemotherapy is therapy that inhibits or kills the proliferation of cancer cells using chemicals, and the toxicity caused by anticancer drugs also occurs in normal cells to exhibit a certain degree of toxicity, and even if anticancer drugs are effective, resistance develops because the effect is lost after a certain period of use. Therefore, there is an urgent need to develop anticancer drugs that act selectively on cancer cells and do not develop resistance. Recently, new anti-cancer drugs targeting the molecular characteristics of cancer have been developed through the acquisition of molecular genetic information about cancer, and there are also reports that anti-cancer drugs targeting characteristic molecular targets only to cancer cells also develop drug resistance. Therefore, the development of a new concept of anticancer agents is required.

Meanwhile, there are experiments using viruses for cancer treatment, due to anecdotal reports of temporary cancer remission after natural viral infection or viral vaccination. The first report was in 1912 due to a reduction in cervical cancer in patients vaccinated against rabies, and similar results were seen in cancer patients following smallpox vaccination or natural viral infections such as mumps or measles. Based on these reports and animal data, patients were first inoculated with live viruses to treat cancer in the late 1940s and early 1950s. However, sometimes, there occurred problems that after temporary tumor reduction, the tumor regrows and the patient dies. These vaccinations did not result in long-term remission. In 1957, Albert B. Sabin, M.D., developed the live oral polio vaccine, mentioned that even when an oncolytic virus kills a tumor, the biggest problem is that the personal immune response to the virus is too fast and the effect is quickly exhausted.

At present, numerous oncolytic adenoviruses have been identified, but to date, the only one virus approved for clinical use anywhere in the world is an Oncorine (H101) subgroup C adenovirus (H101 is a close analog of ONYX015 described by Bischoff et al. in 1996), modified by E1B-55KD deletion that allows conditional replication in P53-deficient cancer cells. Oncorine is administered by intratumoral injection for head and neck cancer. Adenovirus has been widely used not only as a gene delivery vector for gene therapy but also as an oncolytic agent for cancer therapy. The adenovirus exhibits several characteristics suitable for these uses. In other words, the structure and biological properties of the adenovirus have been widely studied, their genomes may be easily modified, and these viruses may infect both replicating and non-replicating cells and may be easily produced in a high titer suitable for clinical use. From a safety aspect, the adenovirus does not cause life-threatening diseases in humans, and its viral genome is non-integrative, preventing insertion mutations. Clinical trials using adenoviral vectors have reported that these viruses have good toxicity and safety profile, although there remains a need for improved efficacy when administered systemically.

Likewise, in the field of gene therapy, systemic administration, i.e., injection into the intravenous or intra-arterial blood flow may also be required to reach a plurality of organs and disseminated cells. For example, in cancer therapy using adenoviral vectors and oncolytic adenoviruses, systemic administration is essential to treat disseminated tumors in an advanced or metastatic state. However, the adenovirus shows significant limitations that reduce a therapeutic effect when injected into the blood flow. Adenovirus type 5 (Ad5) undergoes several neutralizing interactions that drastically reduce the bioavailability of the virus in the blood flow. The biggest problem with the therapy is liver isolation, because > 90% of the injected dose remains in the liver, mainly in liver macrophages, referred to as Kupffer cells, and also in liver sinusoidal endothelial cells (LSECs) and liver cells. Direct interaction between blood cells and proteins is also a major obstacle. Ad5 binds directly to blood cells such as red blood cells through a CAR receptor, and may bind to platelets through integrin. Antibodies not only directly neutralize the virus, but may also trigger innate immune responses by activating complements and docking viral particles with Fc receptors on monocytes and neutrophils. In addition, virus readministration increases the concentration of an anti-Ad neutralizing antibody (NAb), and thus virus neutralization is also enhanced. Opsonization of the adenovirus by the antibody and complement may also enhance clearance by Kupffer cells. Collectively, these interactions result in a significant shortening of the half-life of Ad in blood to about several minutes in mice and humans. Considerable efforts have been made to evade neutralization by antibodies and immune cells during systemic administration of the adenovirus, but systemic propagation is still limited, and it is reported to be temporary and generally ineffective (Ferguson et al. 2012).

Therefore, there is still a need for research on the adenovirus as a cancer therapeutic agent that is suitable for systemic administration and may evade neutralizing antibodies.

### [Disclosure]

### [Technical Problem]

The present invention is directed to provide an antitumor adenovirus.

The present invention is also directed to provide a pharmaceutical composition for treating cancer.

The present invention is further directed to provide a use of the adenovirus for preventing or treating tumors.

The present invention is further directed to provide a method for treating cancer, comprising administering the adenovirus to a subject suffering from cancer.

### [Technical Solution]

In order to achieve the above aspects, the present invention provides an adenovirus comprising a nucleic acid coding a transferrin binding moiety.

The present invention also provides a pharmaceutical composition for treating cancer comprising the antitumor adenovirus.

The present invention also provides a use of the adenovirus for preventing or treating tumors.

The present invention also provides a method for treating cancer, including administering the adenovirus to a subject suffering from cancer.

### [Advantageous Effects]

According to the present invention, the adenovirus comprising a nucleic acid coding for a transferrin binding domain of the present invention exhibits notably increased effects of infecting and killing tumor cells, exhibits increased binding to transferrin, thereby evading an in vivo immune response and thus increasing the plasma half-life, has a systemic therapeutic effect by being specifically delivered to cancer cells, is capable of being topically delivered, and has excellent selectivity, thereby exhibiting the effect of notable antitumor efficacy, and thus may be usefully employed as an anticancer composition or an anticancer adjuvant for various carcinomas.

### [Description of Drawings]

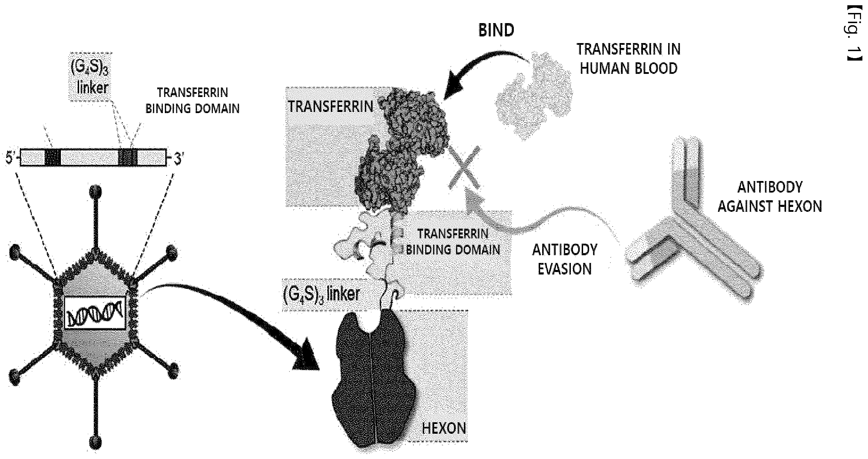

FIG. 1 is a diagram illustrating an antibody evasion mechanism of an antitumor adenovirus comprising a transferrin binding domain of the present invention.
FIG. 2 is a diagram illustrating a process of preparing an antitumor adenovirus comprising a transferrin binding domain (moiety) of the present invention.
FIG. 3 is a diagram illustrating a vector map of an adenovirus envelope-related plasmid pAd1128 comprising the transferrin binding domain of the present invention at an HVR1 position of a hexon.
FIG. 4 is a diagram illustrating a vector map of an adenovirus comprising an albumin binding domain (ABD) (CA10G-A).
FIG. 5 is a diagram illustrating a vector map of an adenovirus comprising a transferrin binding moiety Tbp29aa in a hexon of the adenovirus vector of the present invention (HVR1-Tbp29aa).
FIG. 6 is a diagram illustrating a vector map of an adenovirus comprising a fusion protein containing linkers at the N-terminus and the C-terminus of a transferrin binding moiety Tbp 55aa in the hexon of the adenovirus vector of the present invention (HVR1-Tbp(G₄S₁)₃ 55aa).
FIGS. 7 and 8 are diagrams illustrating immune evasion ability of adenoviruses comprising a transferrin binding domain:
   CA10G: Control adenovirus;
   CA10G-A (CA10G-a): Adenovirus containing an albumin binding domain;
   HVR1-Tbp29aa (Tbp-29aa): Adenovirus containing a transferrin binding moiety Tbp29aa in a hexon of an adenovirus vector; and
   HVR1-Tbp(G₄S₁)₃ 55aa (Tbp(G₄S₁)₃-55aa): Adenovirus including a transferrin binding moiety Tbp55aa and a linker in a hexon of an adenovirus vector.

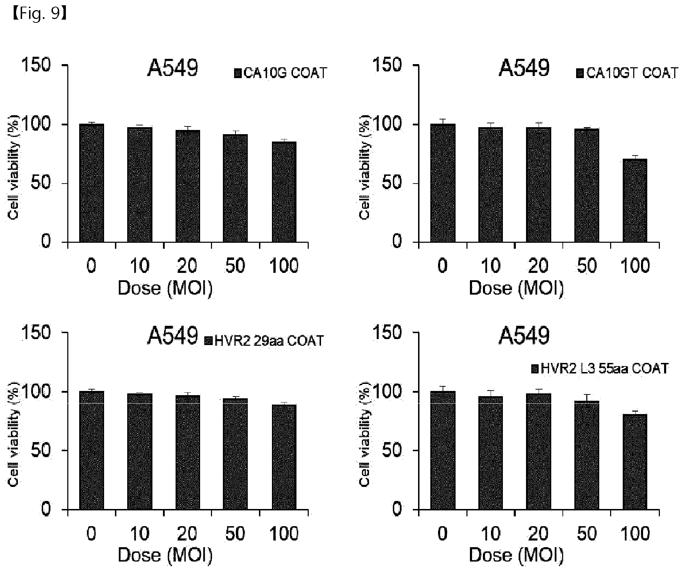

FIG. 9 is a diagram illustrating immune evasion ability according to an insertion position of a transferrin binding moiety into adenoviruses:
   CA10G: Control adenovirus;
   CA10GT: HVR1-(G₄S₁)₃-Tbp 55aa adenovirus with a transferrin binding moiety inserted into an HVR1 position of a hexon;
   HVR2 29aa: HVR2-Tbp 29aa adenovirus with a transferrin binding moiety inserted into an HVR2 position of a hexon; and
   HVR2 L3 55aa: HVR2-(G₄S₁)₃-Tbp 55aa adenovirus.

### [Best Mode of the Invention]

Hereinafter, exemplary embodiments of the present invention will be described in detail. However, the following exemplary embodiments are presented as examples of the present invention, and the present invention is not limited thereby, and various modifications and applications of the present invention may be made within the description of claims to be described below and equivalents interpreted therefrom.

Unless otherwise indicated, a nucleic acid is recorded in a 5'→3' direction from left to right. A numerical range enumerated within the specification is inclusive of numbers defining the range and includes each integer or any non-integer fraction within a defined range.

Unless defined otherwise, all technical and scientific terms used herein have the same meaning as commonly understood by those skilled in the art to which the present invention pertains. Although any methods and materials similar or equivalent to those described herein may be used in the practice for testing the present invention, the preferred materials and methods are described herein.

In one aspect, the present invention relates to an adenovirus comprising a nucleic acid coding a transferrin binding moiety in a coding region of a hypervariable region (HVR) of a hexon protein.

In an exemplary embodiment, the adenovirus may comprise a nucleic acid sequence represented by SEQ ID NO: 1 or 3.

In an exemplary embodiment, the adenovirus may include a nucleic acid sequence of a hexon comprising a transferrin binding moiety, and the nucleic acid sequence of the hexon comprising the transferrin binding moiety may include a base sequence represented by SEQ ID NO: 8 or a base sequence represented by SEQ ID NO: 10, and the hexon comprising the transferrin binding moiety may include an amino acid sequence represented by SEQ ID NO: 9 or 11.

In an exemplary embodiment, the HVR of the hexon protein of the adenovirus may be HVR1, the HVR1 may include an amino acid sequence represented by SEQ ID NO: 7, and the nucleic acid sequence coding HVR1 may include a base sequence represented by SEQ ID NO: 6.

In an exemplary embodiment, the nucleic acid coding the transferrin binding moiety may be included between codons expressing amino acids at positions 154 and 155 of the base sequence (SEQ ID NO: 6) coding the hexon of the adenovirus.

In an exemplary embodiment, the transferrin binding moiety may include an amino acid sequence represented by SEQ ID NO: 2 or 4, and the nucleic acid sequence coding the transferrin binding moiety may include a base sequence represented by SEQ ID NO: 1 or 3.

In an exemplary embodiment, an N-terminus, a C-terminus, or both the N-terminus and the C-terminus of the transferrin binding moiety may be linked to the hexon protein via a linker, and the linker may include an amino acid sequence represented by SEQ ID NO: 5.

In an exemplary embodiment, the transferrin binding moiety may be attached directly to the hexon protein, and in other words, the N-terminus and the C-terminus of the transferrin binding moiety are linked directly to the hexon protein. However, the transferrin binding moiety may also be linked to the hexon protein via a linker sequence. Accordingly, in another exemplary embodiment, the N-terminus and/or C-terminus of the transferrin binding moiety are linked to the hexon protein via a linker sequence.

In an exemplary embodiment, when the hexon protein of the adenovirus is assembled into a capsid, the transferrin binding moiety may be located on the outer surface of the hexon protein.

The adenovirus of the present invention may be coated with a transferrin binding domain by including the transferrin binding moiety on the outer surface of the hexon protein, thereby protecting the adenovirus itself from neutralizing antibodies present in the blood flow.

In an exemplary embodiment, the adenovirus may be a human adenovirus, may be selected from the group consisting of human adenovirus serotypes 1 to 57, and may be a human adenovirus serotype 5 (GenBank: AY339865.1), and may be a chimeric adenovirus of a human adenovirus serotype 5/3.

In an exemplary embodiment, the adenovirus may further include a tissue-specific promoter or a tumor-specific promoter, and the promoter may be operably linked to an endogenous gene of the adenovirus.

In an exemplary embodiment, the promoter may be selected from the group consisting of an E2F promoter, a telomerase hTERT promoter, a tyrosinase promoter, a prostate-specific antigen promoter, an alpha-fetoprotein promoter, and a COX-2 promoter.

In an exemplary embodiment, the telomerase hTERT promoter may include a base sequence represented by SEQ ID NO: 17 and may be operably linked to E1A and E1B of the endogenous gene of the adenovirus.

In an exemplary embodiment, the endogenous gene of the adenovirus has a structure of 5'-ITR-C1-C2-C3-C4-C5 3-'ITR; wherein the C1 may include E1A, E1B or E1A-E1B; the C2 may include E2B-L1-L2-L3-E2A-L4; the C3 may include E3 or not; the C4 may include L5; and the C5 may include E4 or not.

In an exemplary embodiment, an IRES sequence may be further included between E1A and E1B of the endogenous gene of the adenovirus.

In an exemplary embodiment, the E1A may include a base sequence represented by SEQ ID NO: 18.

In an exemplary embodiment, the E1B may include a base sequence represented by SEQ ID NO: 19.

In an exemplary embodiment, the IRES may include a base sequence represented by SEQ ID NO: 20.

In an exemplary embodiment, the promoter may be operably linked to E1A and E1B of the endogenous gene of the adenovirus.

In an exemplary embodiment, the adenovirus may include hTERT promoter-E1A-IRES-EIB including a base sequence represented by SEQ ID NO: 15.

In an exemplary embodiment, the adenovirus may further include an expression cassette that expresses a foreign gene, and the expression cassette may be included in the E3 region of the endogenous gene of the adenovirus.

In an exemplary embodiment, the adenovirus of the present invention may further include a CMV promoter and a foreign gene, and may include the CMV promoter and a foreign gene operably linked thereto in the E3 region of the endogenous gene of the adenovirus.

In an exemplary embodiment, the adenovirus may be an antitumor adenovirus, which is an oncolytic adenovirus.

In an exemplary embodiment, the adenovirus of the present invention may have a higher tumor killing ability than a wild-type adenovirus and may be an oncolytic adenovirus.

In an exemplary embodiment, the adenovirus of the present invention is a replication-competent adenovirus, antitumor or oncolytic adenovirus.

In other exemplary embodiment, the adenovirus is a replication-incompetent adenovirus or replication-deficient adenovirus. The replication-deficient adenovirus or replication-incompetent adenovirus is an adenovirus that is unable to replicate in a target cell and used in gene therapy as a gene carrier to a target cell due to the purpose to express a therapeutic gene within the cell and not to degrade the cell.

In an exemplary embodiment, the adenovirus may further include a capsid modification to increase the infectivity of the adenovirus or to be targeted to a receptor present in a tumor cell.

In an exemplary embodiment, the capsid modification may be insertion of an RGD motif within an H1 loop of an adenovirus fiber protein.

In an exemplary embodiment, the capsid modification may be substitution of a fiber gene or a part thereof with a homologous part derived from another serotype of adenovirus to form a chimeric adenovirus, and may be prepared to include a capsid substituted with a fiber gene derived from a serotype 3 adenovirus or a part thereof and include a serotype 5 adenovirus-derived gene in a portion excluding the fiber gene.

In an exemplary embodiment, the adenovirus may include one or more non-adenoviral genes, and the non-adenoviral genes may be genes used in cancer gene therapy, and the genes used in cancer gene therapy may be selected from the group consisting of tumor-suppressor genes, genes coding anti-tumor interfering RNA, and immunostimulatory genes.

The adenovirus of the present invention may be selectively distributed to a predetermined tissue in vivo, thereby evading or significantly reducing expression in non-target or non-tumor tissues.

In an exemplary embodiment, the antitumor adenovirus of the present invention may be used in cancer gene therapy.

In the present invention, the term "transferrin" is a glycoprotein that binds to iron very strongly and reversibly. Although only about 0.1% of iron in the body binds to transferrin, the transferrin plays a very important role in regulating the amount of iron in the body. The transferrin exists in the blood and plays a role in absorbing iron. In the present invention, blood transferrin binds to the transferrin binding moiety of the present invention to allow the adenovirus to evade antibodies in vivo, thereby increasing the blood half-life of the adenovirus when administered systemically.

In the present invention, the term "transferrin binding moiety" refers to any amino acid sequence that is capable of binding to transferrin, that is, has binding affinity. Preferably, the transferrin binding moiety may bind to serum transferrin, and more preferably human serum transferrin. The term "transferrin binding moiety" includes a natural transferrin binding domain (e.g., transferrin present in bacterial proteins) and a synthetic peptide-derived transferrin-binding sequence. In a preferred exemplary embodiment, the transferrin binding moiety is selected from a transferrin binding domain from *Neisseria Meningitidis,* and functionally equivalent variants thereof. The term "transferrin binding domain" refers to any region derived from a native protein capable of binding to transferrin with sufficient specificity to ensure protection from neutralizing antibodies, but in the present invention, may be used herein synonymously with the transferrin binding moiety. In the present invention, the transferrin binding moiety, the transferrin binding domain, and the transferrin binding peptide/protein are used interchangeably. However, the transferrin binding moiety includes both the transferrin binding domain and the transferrin binding peptide/protein, and more preferably, the transferrin binding moiety may refer to a synthetic peptide comprising some amino acid sequences of the transferrin binding domain of the transferrin binding peptide/protein.

In addition, the present invention includes functionally equivalent variants of the transferrin binding moiety described above. As used herein, the term "functionally equivalent variant" refers to any polypeptide that is derived from the transferrin binding moiety by insertion, deletion, or substitution of one or more residues and substantially retains the ability to interact with transferrin as determined above. In a preferred exemplary embodiment, if the polypeptide has at least 10%, 20%, 30%, 40%, 50%, 60%, 70%, 80%, 90% or 100% of transferrin binding ability of the transferrin binding domain of SEQ ID NO: 2 or 4, the polypeptide is considered a functionally equivalent variant of the transferrin binding moiety. Preferably, if the polypeptide may neutralize the antibodies with at least 10%, 20%, 30%, 40%, 50%, 60%, 70%, 80%, 90% or 100% of efficiency with the transferrin binding domain of SEQ ID NO: 2 or 4, the polypeptide is considered a functionally equivalent variant of the transferrin binding moiety. The level of identity between the two polypeptides is determined using computer algorithms and methods well known to those skilled in the art. The identity between two amino acid sequences is preferably determined using the BLASTP algorithm [BLAST Manual, Altschul, S., et al., NCBI NLM NIH Bethesda, Md. 20894, Altschul, S., et al., J. Mol. Biol. 215: 403-410 (1990)], but may also be determined using other similar algorithms. Sequence identity % is determined using BLAST and BLAST 2.0 by applying the parameters mentioned herein. Software for performing BLAST analysis is publicly available through the National Center for Biotechnology Information.

In the present invention, the types of transferrin binding moiety include various peptides, antibodies, etc. in addition to the moieties described in the exemplary embodiment of the present invention, but are not limited thereto. The transferrin binding moiety includes transferrin binding proteins (Tbp1, Tbp2, TbpA, TbpB, etc.) derived from various bacteria, iron-regulated proteins, iron-regulated outer membrane proteins, transferrin receptor proteins, an outer membrane protein B1 of *Moraxella catarrhalis,* chimeric fusion proteins containing a transferrin binding peptide, a recombinant transferrin binding peptide, fragments of the peptide or protein or analogs thereof, transferrin-binding molecules, or anti-transferrin antibodies or immunologically active fragments thereof. The transferrin binding moiety binding to the adenovirus may be applied to the present invention as long as the moiety binds to transferrin in the blood to evade an in vivo immune response.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment produced by gene fusion (WO94/13804).

In the present invention, the term "linker sequence" refers to an amino acid sequence that acts as a hinge between the hexon protein and the transferrin binding moiety to provide a space between the two elements, so that the secondary structure of the hexon protein does not influences the presence of the transferrin binding moiety and vice versa. The linker sequence may have any length that allows two elements to move independently of each other while maintaining a tertiary structure of individual elements. In a preferred exemplary embodiment, the linker sequence is a flexible linker peptide having less than 31 amino acids in length. More preferably, the linker sequence contains less than 10, less than 5, less than 4, or less than 2 amino acids. In an exemplary embodiment, the linker sequence includes two or more amino acids selected from the group consisting of glycine, serine, alanine, and threonine. In another exemplary embodiment, the linker is a polyglycine linker. In the present invention, the linker sequence may be represented by SEQ ID NO: 5. Other linkers may also be used alternatively (Reddy Chichili, VP., Kumar, V., and Sivaraman, J. (2013). Linkers in the structural biology of protein-protein interactions. Protein Science 22(2):153-67).

In the present invention, the term "operably linked" means a functional linkage between a gene expression regulatory sequence (e.g., an array of a promoter, a signal sequence, or a transcriptional regulatory factor binding site) and another gene sequence, and the regulatory sequence regulates the transcription and/or translation of the another gene sequence.

In the present invention, the term "promoter" refers to an untranslated nucleic acid sequence upstream of a coding region that includes a binding site for RNA polymerase and has transcription initiation activity into mRNA of a downstream promoter gene. In the expression cassette of the present invention, the promoter may be any promoter capable of initiating expression of shRNA. Specifically, the promoter of the present invention may be used with a promoter that constantly induces the expression of a target gene at all times (a constitutive promoter) or a promoter that induces the expression of the target gene at specific location and time (an inducible promoter). Examples thereof include a U6 promoter, a H1 promoter, a cytomegalovirus (CMV) promoter, a SV40 promoter, a CAG promoter (Hitoshi Niwa et al., Gene, 108:193-199, 1991), a CaMV 35S promoter (Odell et al., Nature 313: 810-812, 1985), an Rsyn7 promoter (US Patent Application No. 08/991,601), a rice actin promoter (McElroy et al., Plant Cell 2:163-171, 1990), a ubiquitin promoter (Christensen et al.., Plant Mol. Biol. 12:619-632, 1989), an ALS promoter (US Patent Application No. 08/409,297), etc. In addition, promoters, etc. disclosed in US Patent Registration Nos. 5,608,149; 5,608,144, 5,604,121, 5,569,597, 5,466,785, 5,399,680, 5,268,463, 5,608,142, etc. may be used with all known promoters well-known to those skilled in the art, but are not limited thereto. Preferably, the promoter of the present invention may be a U6 promoter, a HI promoter, or a CMV promoter, and according to a preferred exemplary embodiment of the present invention, the CMV promoter may be used.

In the present invention, the term "tissue-specific" is intended to mean that a promoter to which a gene required for replication is operably linked functions tissuespecifically to replicate in the tissue. This may occur because a positive transcription factor that activates the promoter is present in the tissue and not present in a non-target tissue. In addition, this may occur due to the absence of transcriptional inhibitory factors that are normally formed in a non-target tissue to prevent transcription as a result of the promoter. Accordingly, when the transcription occurs, the transcription is directed toward genes required for replication, so that replication of the vector and its accompanying functions may occur in the target tissue.

Tissue specificity is specifically related to targeting an abnormal counterpart of a particular tissue while evading a normal counterpart of the tissue, or evading other types of surrounding tissues other than the abnormal tissue while treating the abnormal tissue. In a specific exemplary embodiment, the promoter is "tumor-specific," which means that the promoter functions specifically in tumor tissue. For example, the recombinant adenovirus of the present invention is useful for treating metastasis to the liver. A specific example is colon cancer, which often metastasizes to the liver. When the colon cancer metastasizes to the liver, it has been confirmed that a CEA promoter is active in metastatic cells, but not in normal liver cells. Accordingly, normal human adult liver will not support replication of a virus with replicationessential viral genes linked to a colon cancer CEA-specific promoter. The replication needs to occur in a primary cancer cell. Another example is an alpha-fetoprotein promoter, which has activity only in hepatocellular carcinoma. Another example is a tyrosinase promoter, which exhibits the activity only in melanoma and does not exhibit the activity in normal skin. In each case, the replication is expected in abnormal cells, but not in normal cells.

Examples of the tissue-specific promoter include, unrestrictively, an alpha-fetoprotein promoter, a DE3 promoter, a tyrosinase promoter, a carcinoembryonic antigen (CEA) promoter, a surfactant protein promoter, an E2F promoter, a telomerase hTERT promoter, a prostate-specific antigen promoter, a COX-2 promoter, an albumin gene promoter, a core promoter of a hepatitis virus, and a promoter of thyroxine and a globulin-binding protein binding to an ErbB2 promoter.

In the present invention, the term "adenovirus" refers to any virus that may be classified as an adenovirus, that is, any virus belonging to the family Adenoviridae, which is characterized as a non-enveloped virus with an icosahedral nucleocapsid containing a double-stranded DNA genome. The term includes any adenovirus capable of infecting humans or animals, including all groups, subgroups and serotypes that utilize CAR as a receptor for infecting target cells. The adenovirus of the present invention include, as a non-limiting example, avian, canine, horse, bovine, sheep, porcine, human or frog adenoviruses. In a preferred exemplary embodiment, the adenovirus of the present invention is a human adenovirus, i.e., an adenovirus capable of infecting humans. According to the present invention, the "serotypes" are immunologically different types of adenovirus. In the case of a human adenovirus, there are at least 57 serotypes classified into several subgroups A to G.

The adenovirus of the present invention is a recombinant adenovirus. In the present invention, the term "recombinant" refers to an adenovirus that is not produced naturally. The recombinant adenovirus includes one or more modifications compared to a wild type. Such modifications include, non-restrictively, modifications to the adenovirus genome that is packed into a particle to produce infectious virus. In other modifications, replication-deficient viruses (i.e., viruses unable to reproduce) may be obtained by removing genes important for replication from the viral genome. Examples of the modification include deletions known in the art, such as deletion and the like of one or more of the E1a, E1b, E2a, E2b, E3 or E4 coding regions. Other modifications include deletions of the whole coding region of the adenovirus genome. These adenoviruses are called "gutless" adenoviruses. These adenoviruses also include chimeric adenoviruses prepared by combining factors derived from different serotypes. An adenovirus particle consists of a capsid that encapsulates viral DNA. As used herein, the term "capsid is a viral protein shell formed by subunits called capsomers, which may be pentagonal or hexagonal. The adenovirus capsid has an icosahedral shape with 20 equilateral triangular faces. Most of the capsid is formed by the hexon protein, and each vertex is a complex formed by a penton base and a fiber protein.

The adenovirus of the present invention may have modifications in the genome sequence, which impart selective replication in cells. To apply the adenovirus expression to a tissue requiring the expression of the adenovirus or to a tumor tissue to be treated, the adenovirus of the present invention may include a tissue-specific promoter or tumor-specific promoter. Accordingly, in an exemplary embodiment, the adenovirus further includes a tissue-specific promoter or tumor-specific promoter.

As used herein, the term "recombinant" also includes a replication-conditional adenovirus, which replicates preferentially in a specific type of cell or tissue, but replicates less or not at all even in other types. For example, among the adenoviruses presented in the present invention, the recombinant adenovirus is an adenovirus that replicates in abnormally proliferating tissues, such as solid tumors and other neoplasms. These adenoviruses include viruses mentioned in US Patent 5,998,205 and US Patent 5,801,029. These viruses are sometimes referred to as "cytolytic" or "cytopathic" viruses (or vectors), and when the viruses have these effects on neoplastic cells, the viruses are called "oncolytic" viruses (or vectors).

In the present invention, the term "adenovirus hexon protein" or "hexon protein" (conventionally referred to as "protein II") refers to a capsid-main structural protein found in an adenovirus that self-assembles to form trimers having a hexagonal shape. 240 hexon trimers are assembled to form an adenovirus capsid. The hexon protein is essential for viral capsid assembly, determination of the icosahedral symmetry of the capsid, and capsid integrity. Although the major structural features of the hexon protein are common among adenovirus serotypes, the size and immunological properties of the hexon protein have differences between the serotypes.

In the present invention, the term "hexon protein" includes, as a non-limiting example, any hexon protein of adenovirus, such as a protein defined by a sequence in the UniProt database with accession number P04133 registered on February 19, 2014, corresponding to a hexon protein of human adenovirus C serotype 5; a protein defined by a sequence in the UniProt database with accession number P03277 registered on February 19, 2014, corresponding to a hexon protein of human adenovirus C serotype 2; a protein defined by a sequence in the UniProt database with accession number P42671 registered on February 19, 2014, corresponding to a hexon protein of avian adenovirus gall (strain Phelps); and a protein defined by a sequence in the UniProt database with accession number P11819 registered on February 19, 2014, corresponding to a hexon protein of human adenovirus F serotype 40. This expression includes all natural variants of the hexon proteins that occur naturally in different subgroups or serotypes.

In the present invention, the expression "outer surface of the hexon protein" refers to a region exposed on the surface of the capsid of the hexon protein. In order to determine whether the transferrin binding moiety of the present invention has been introduced into the inner part or the outer surface of the adenovirus hexon protein, an assay for detecting binding to human serum transferrin may be performed as described in the experimental section of the present application (e.g., ELISA assay) or by an in vitro neutralization assay. If the human serum transferrin may bind to the adenovirus, the transferrin binding moiety has been introduced to the outer surface of the adenovirus hexon protein.

It is reported that Loop 1 (L1) and Loop 2 (L2) of the hexon protein are exposed on the outer surface of a viral capsomere structure. L1 includes six hypervariable regions (HVRs), that is, HVR1 to HVR6, and L2 includes the seventh hypervariable region (HVR7). As used herein, the term "hypervariable region" or "HVR" refers to a region in the surface-exposed loops that differs in length and sequence that determine the serotype of the adenovirus. Each subunit of the trimer has seven hypervariable regions of the adenovirus hexon. In the present invention, the names used for HVR are described in Crawford-Miksza and Schnurr (Crawford-Miksza and Schnurr. 1996. Virology, 224(2):357-367). In a preferred exemplary embodiment of the present invention, the HVR is HVR1, but is not limited thereto. The sequence coding the transferrin binding moiety is inserted so that the prepared fusion protein includes the transferrin binding moiety after amino acid D154 of the hexon protein, according to the numbering of a hexon protein in GenBank accession number BAG48782.1 on June 14, 2008, which corresponds to the hexon protein derived from human adenovirus serotype 5.

In the present invention, the adenovirus is an oncolytic adenovirus, and preferably, an adenovirus further including mutations in one or more genes selected from the group consisting of E1a, E1b, E4, and VA-RNA to achieve selective replication of the adenovirus genome in tumors.

In another exemplary embodiment, the adenovirus genome further includes capsid modifications to increase adenovirus infectivity or to be targeted by receptors present on tumor cells. Preferably, the capsid modification is insertion of an RGD motif into the H1 loop of the adenovirus fiber protein. In another exemplary embodiment, the adenovirus genome is a chimeric adenovirus genome derived from one predetermined serotype, including fragments or regions of the genome substituted with a homologous region of another serotype-derived genome. Preferably, the chimeric adenovirus is a human adenovirus derived from another serotype, preferably serotype 5 including a part of a fiber gene substituted with a homologous region derived from a human adenovirus 3 or human adenovirus 35. In a preferred exemplary embodiment, the capsid modification is substitution of a part of the fiber gene with a homologous portion derived from another adenovirus serotype to form a chimeric adenovirus. In the present invention, adenovirus type 5/3 includes a part of the fiber gene derived from adenovirus type 3, and other genomes refer to viruses derived from adenovirus type 5.

In another exemplary embodiment, the adenovirus genome includes an additional gene inserted into the genome. In an exemplary embodiment, the gene is used for gene therapy or vaccine immunization. Preferably, the gene is a gene used for cancer gene therapy, and more preferably a gene selected from the group consisting of at least a prodrug-activating gene, a tumor-suppressor gene, a gene coding antitumor interfering RNA, and an immunostimulatory gene.

In one aspect, the present invention relates to a pharmaceutical composition for treating cancer including the adenovirus of the present invention as an active ingredient.

In a preferred exemplary embodiment, the adenovirus of the present invention is an oncolytic adenovirus. As used herein, the term "oncolytic adenovirus" refers to any adenovirus that is replication-competent or capable of replicating in a tumor, even without selectivity. The therapeutic action of the oncolytic adenovirus is based on the ability to cytolyze tumor cells that are to replicate and be eliminated. The killing of tumor cells may be detected by any latest method, such as measurement of the number of viable cells, cytopathic effects, apoptosis of tumor cells, viral protein synthesis in tumor cells (e.g., metabolic labeling, Western blotting of viral proteins, or PCR using reverse transcription of viral genes required for replication), reduction in tumor size, or the like.

In an exemplary embodiment, the composition of the present invention may be administered systemically.

In an exemplary embodiment, the composition of the present invention may be used for intravenous administration.

In an exemplary embodiment, the composition of the present invention may further include an anticancer agent. For example, the anticancer agent may include acivicin, aclarubicin, acodazole, acronycine, adozelesin, alanosine, aldesleukin, allopurinol sodium, altretamine, aminoglutethimide, amonafide, ampligen, amsacrine, androgens, anguidine, aphidicolin glycinate, asaley, asparaginase, 5-azacitidine, azathioprine, Bacillus calmette-guerin (BCG), Baker's Antifol, beta-2'-deoxythioguanosine, bisantrene HCL, bleomycin sulfate, busulfan, buthionine sulfoximine, BWA 773U82, BW 502U83/HCl, BW 7U85 mesylate, ceracemide, carbetimer, carboplatin, carmustine, chlorambucil, chloroquinoxaline-sulfonamide, chlorozotocin, chromomycin A3, cisplatin, cladribine, corticosteroids, Corynebacterium parvum, CPT-11, crisnatol, cyclocytidine, cyclophosphamide, cytarabine, cytembena, dabis maleate, dacarbazine, dactinomycin, daunorubicin HCl, deazauridine, dexrazoxane, dianhydrogalactitol, diaziquone, dibromodulcitol, didemnin B, diethyldithiocarbamate, diglycoaldehyde, dihydro-5-azacytidine, doxorubicin, echinomycin, edatrexate, edelfosine, eflomithine, Elliott's solution, elsamitrucin, epirubicin, esorubicin, estramustine phosphate, estrogens, etanidazole, ethiofos, etoposide, fadrazole, fazarabine, fenretinide, filgrastim, finasteride, flavone acetic acid, floxuridine, fludarabine phosphate, 5-fluorouracil, Fluosol^{™}, flutamide, gallium nitrate, gemcitabine, goserelin acetate, hepsulfam, hexamethylene bisacetamide, homoharringtonine, hydrazine sulfate, 4-hydroxyandrostenedione, hydrozyurea, idarubicin HCl, ifosfamide, 4-ipomeanol, iproplatin, isotretinoin, leucovorin calcium, leuprolide acetate, levamisole, liposomal daunorubicin, liposome encapsulated doxorubicin, lomustine, lonidamine, maytansine, mechlorethamine hydrochloride, melphalan, menogaril, merbarone, 6-mercaptopurine, mesna, methanol extract of Bacillus calmette-guerin, methotrexate, N-methylformamide, mifepristone, mitoguazone, mitomycin-C, mitotane, mitoxantrone hydrochloride, monocyte/macrophage colony-stimulating factor, nabilone, nafoxidine, neocarzinostatin, octreotide acetate, ormaplatin, oxaliplatin, paclitaxel, pala, pentostatin, piperazinedione, pipobroman, pirarubicin, piritrexim, piroxantrone hydrochloride, PIXY-321, plicamycin, porfimer sodium, prednimustine, procarbazine, progestins, pyrazofurin, razoxane, sargramostim, semustine, spirogermanium, spiromustine, streptonigrin, streptozocin, sulofenur, suramin sodium, tamoxifen, taxotere, tegafur, teniposide, terephthalamidine, teroxirone, thioguanine, thiotepa, thymidine injection, tiazofurin, topotecan, toremifene, tretinoin, trifluoperazine hydrochloride, trifluridine, trimetrexate, tumor necrosis factor (TNF), uracil mustard, vinblastine sulfate, vincristine sulfate, vindesine, vinorelbine, vinzolidine, Yoshi 864, zorubicin, cytosine arabinoside, etoposide, melphalan, taxol, and mixtures thereof. The anticancer agent may be preferably cisplatin, paclitaxel, 5-fluorouracil (5-FU), methotrexate, doxorubicin, daunorubicin, cytosine arabinoside, etoposide, melphalan, chlorambucil, cyclophosphamide, vindesine, mytomycin, bleomycin, tamoxifen, and taxol, and more preferably cisplatin, paclitaxel, or 5-fluorouracil (5-FU). The anticancer agent is not limited thereto, so long as the anticancer agent achieves the purpose of showing a synergistic effect on anticancer effects by treatment in combination with the composition of the present invention.

In an exemplary embodiment, the cancer may be at least one selected from the group consisting of colorectal cancer, breast cancer, uterine cancer, cervical cancer, ovarian cancer, prostate cancer, brain tumor, head and neck carcinoma, melanoma, myeloma, leukemia, lymphoma, gastric cancer, lung cancer, pancreatic cancer, nonsmall cell lung cancer, liver cancer, esophageal cancer, small intestine cancer, perianal cancer, fallopian tube carcinoma, endometrial carcinoma, vaginal carcinoma, vulvar carcinoma, Hodgkin's disease, bladder cancer, kidney cancer, ureteral cancer, renal cell carcinoma, renal pelvic carcinoma, bone cancer, skin cancer, head cancer, cervical cancer, skin melanoma, intraocular melanoma, endocrine cancer, thyroid cancer, parathyroid cancer, adrenal cancer, soft tissue sarcoma, urethral cancer, penile cancer, central nervous system (CNS) tumor, primary CNS lymphoma, spinal cord tumor, brainstem glioma, and pituitary adenoma.

The composition of the present invention may further include an adjuvant. The adjuvant may be used with any adjuvant known in the art without limitation, but further include, for example, a Freund's complete adjuvant or an incomplete adjuvant to increase the effect thereof.

The composition according to the present invention may be prepared in the form of incorporating the active ingredient into a pharmaceutically acceptable carrier. Here, the pharmaceutically acceptable carrier includes carriers, excipients and diluents commonly used in a pharmaceutical field. The pharmaceutically acceptable carrier that may be used in the composition of the present invention is not limited thereto, but may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil.

The composition according to the present invention may be formulated and used in the form of oral formulations such as powders, granules, tablets, capsules, suspensions, emulsions, syrups, aerosols, etc., external preparations, suppositories, and sterile injectable solutions according to a conventional method.

The formulations may be prepared by using diluents or excipients, such as a filler, an extender, a binder, a wetting agent, a disintegrating agent, a surfactant, etc., which are generally used. Solid formulations for oral administration include tablets, pills, powders, granules, capsules, etc., and these solid formulations may be prepared by mixing at least one excipient, for example, starch, calcium carbonate, sucrose, lactose, gelatin, etc. with the active ingredients. Further, lubricants such as magnesium stearate and talc may be used in addition to simple excipients. Liquid formulations for oral administration may correspond to suspensions, oral liquids, emulsions, syrups, etc., and may include various excipients, for example, a wetting agent, a sweetener, an aromatic agent, a preserving agent, etc., in addition to the commonly used simple diluents, such as water and liquid paraffin. Formulations for parenteral administration include sterile aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, etc. may be used. As the base of the suppository, witepsol, Tween 61, cacao butter, laurinum, glycerogelatin, etc. may be used.

The composition of the present invention may be administered to a subject through various routes. All methods of administration may be expected, and the composition may be administered, for example, oral, intravenous, intramuscular, subcutaneous, and intraperitoneal injection, but most preferably intravenous administration.

The dose of the pharmaceutical composition according to the present invention is selected in consideration of the age, body weight, sex, and physical conditions of the subject. It is obvious that the concentration of the single domain antibody included in the pharmaceutical composition may be variously selected according to a subject, and preferably included in the pharmaceutical composition at a concentration of 0.01 to 5,000 µg/ml. When the concentration is less than 0.01 µg/ml, pharmaceutical activity may not be shown, and when the concentration exceeds 5,000 µg/ml, toxicity to the human body may be exhibited.

The composition of the present invention may be used to prevent or treat cancer and its complications, and may also be used as an anticancer adjuvant.

The composition of the present invention is administered in a therapeutically effective amount or pharmaceutically effective amount. The term "pharmaceutically effective amount" refers to an amount enough to treat diseases at a reasonable benefit/risk ratio applicable to medical treatment, and the effective amount level may be determined according to factors including the type, severity, age, and sex of a subject, the activity of a drug, the sensitivity to a drug, a time of administration, a route of administration, an excretion rate, duration of treatment, and drugs to be simultaneously used, and other factors well-known in the medical field.

In one aspect, the present invention relates to a use of an adenovirus including a nucleic acid coding the transferrin binding moiety of the present invention in a coding region of a hypervariable region (HVR) of a hexon protein for prevention or treatment of tumors.

In one aspect, the present invention relates to a method for treating cancer including administering an adenovirus including a nucleic acid coding the transferrin binding moiety of the present invention in a coding region of a hypervariable region (HVR) of a hexon protein to a subject suffering from cancer.

### [Modes of the Invention]

Hereinafter, the present invention will be described in more detail with reference to the following Examples. However, the following Examples are only intended to embody the contents of the present invention, and the present invention is not limited thereto.

### Example 1. Preparation of anticancer (antitumor) virus including transferrin binding domain

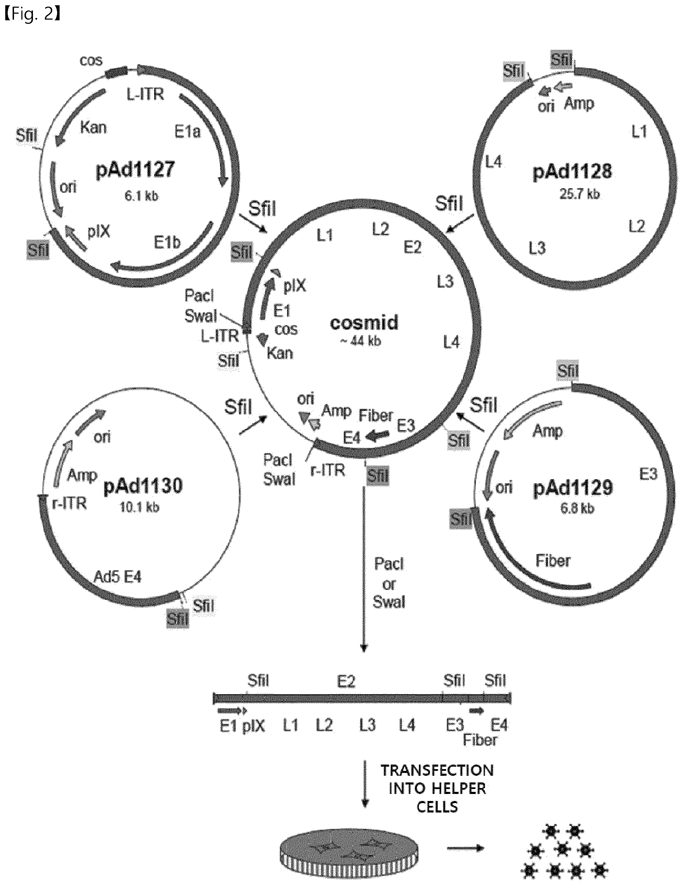

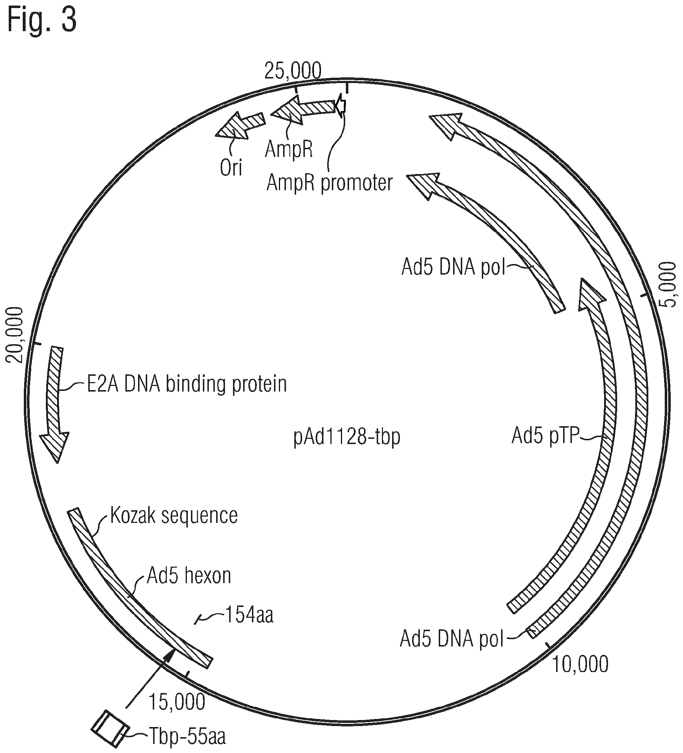

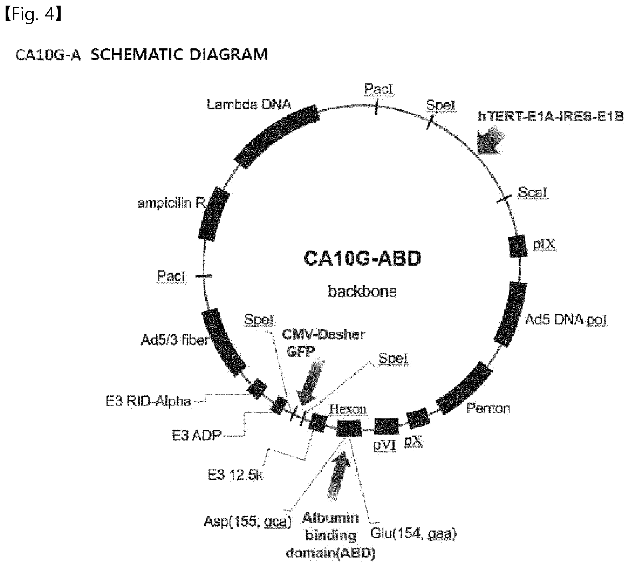

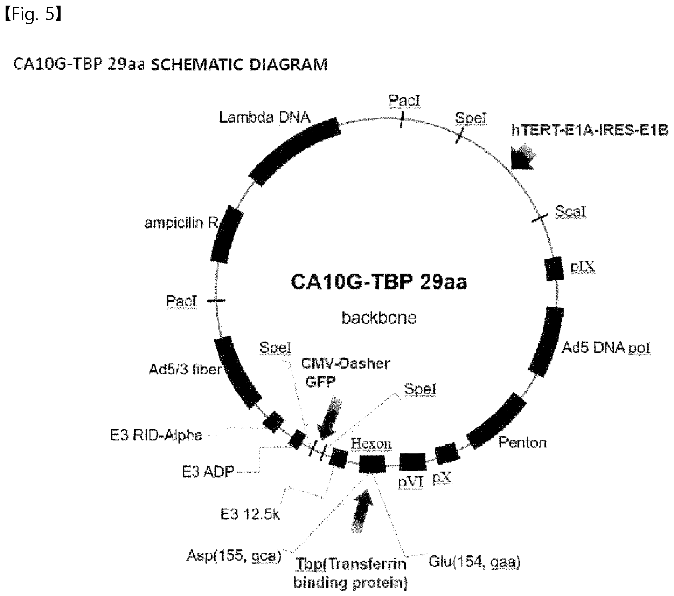

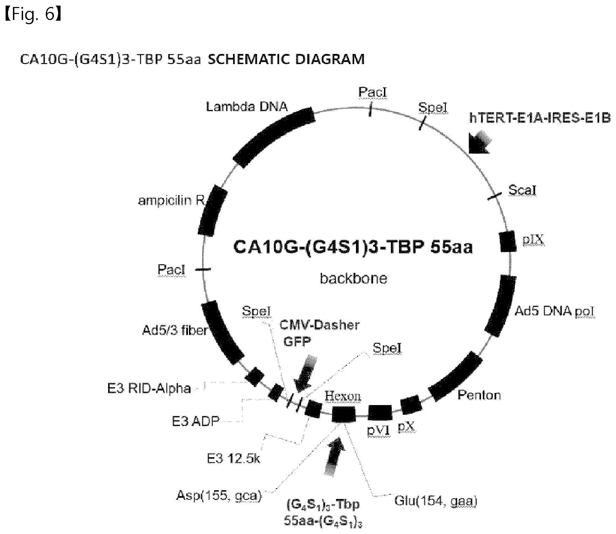

An antitumor adenovirus including a transferrin binding moiety was prepared by inserting base sequences (SEQ ID NOs: 1 and 3) coding two types of transferrin binding moieties Tbp29aa (SEQ ID NO: 2) and Tbp55aa (SEQ ID NO: 4) from transferrin binding domains (TBDs) of transferrin binding proteins (TBPs) derived from *Neisseria Meningitidis* strains K454, S3131, and B16B6 into an HVR1 region of a hexon of an adenovirus vector, respectively. Specifically, the synthesis was requested to Bio Basic Inc. with respect to a base sequence (SEQ ID NO: 1) coding Tbp29aa and a base sequence coding a fusion protein including a linker (SEQ ID NO: 5) at the N-terminus and the C-terminus of Tbp55aa (SEQ ID NO: 4) after designing two types of transferrin binding moieties, Tbp29aa (SEQ ID NO: 2) and Tbp55aa (SEQ ID NO: 4) in a transferrin binding domain by referring to amino acids of TbpA of *Neisseria Meningitidis* strains K454, S3131, and B16B6. After synthesis, each target sequence was amplified by a PCR method, and then inserted to an HVR1 position (between codons expressing amino acid (gaa, E) at position 154 and amino acid (gac, D) at position 155) of a base sequence (SEQ ID NO: 14) coding a hexon of a pAd1128 plasmid (OD260, adenoviral plasmid) and transformed into *E.Coli*DHlOB. Thereafter, clones in which the insertion of each transferrin binding moiety was confirmed through colony PCR were mini-prepped, and then the base sequences were confirmed again by requesting sequencing from macrogen. Thereafter, in order to prepare an adenovirus genome (comid) to be used as a material for producing an adenovirus, four plasmids of the pAd1128-tbp (including each transferrin binding moiety coding sequence), pAd1127 (including E and PIX sites), pAd1129 (including E3 and Fiber)-GFP and pAd1130 (including E4) were treated with a restriction enzyme Sfil and then ligated to prepare a cosmid (FIG. 2). Thereafter, the cosmid was packaged into lambda phage and then transduced by infection in *E.Coli* (OD101). Several clones were liquid-cultured and mini-prepped, and then treated with the restriction enzyme, and the size of a DNA fragment was compared with an expected pattern, and clones with the expected pattern were taken. Thereafter, the confirmed clones were mini-prepped and treated with the restriction enzyme to remove an origin of replication, an antibiotic resistance gene, etc. to prepare a linear adenovirus genome, and then 7 µg of the linear adenovirus genome was transduced into a HE293 cell line to prepare a seed virus, and the seed virus was used for the experiment later. A schematic diagram of a vector of the seed virus envelope was shown in FIG. 3, and a vector structure of the adenovirus including the transferrin binding moiety Tbp29aa was illustrated in FIG. 5 (adenovirus including SEQ ID NO: 8; HVR1-TBP 29aa), and a vector structure of the adenovirus including Tbp55aa including linkers at the N-terminus and the C-terminus was illustrated in FIG. 6 (adenovirus including SEQ ID NO: 10; HVR1-(G₄S₁)₃-TBP 55aa). In addition, a vector structure of adenovirus type 5/3 CA10G-A (adenovirus including a sequence represented by SEQ ID NO: 12) including an albumin binding domain (ABD) in a HVR1 region to be used as a positive control was illustrated in FIG. 4.

### Example 2. Confirmation of neutralizing antibody evasion ability of antitumor virus

It was confirmed whether the adenovirus including the transferrin binding domain prepared in Example 1 bound to transferrin in vivo to evade an antibody attack and kill cancer cells. Specifically, 100 µl of a lung cancer cell line A549 was dispensed into a 96-well plate to be 1 × 10⁴ cells per well. The positive control, CA10G-A (adenovirus type 5/3 including the albumin binding moiety in the HVR1 region: adenovirus including the sequence represented by SEQ ID NO: 12) was ligated at 4°C for 4 hours in a culture medium containing 10 mg/ml of albumin before cell treatment to evade the antibody. In the present invention, HVR1-TBP 29aa (adenovirus type 5/3 including a transferrin binding moiety represented by SEQ ID NO: 1 in the HVR1 region) and HVR1-(G₄S₁)₃-TBP 55aa (adenovirus type 5/3 including a transferrin binding moiety represented by SEQ ID NO: 3 in the HVR1 region and a linker) were ligated at 4°C for 4 hours in a culture medium containing 1 mg/ml of transferrin before cell treatment. Thereafter, the virus reacted with albumin or transferrin and the control virus (CA10G: adenovirus type 5/3) (infectious units, IFU) were treated at 0, 5, 10, 20, 50, and 100-fold the cell number, respectively, and an adenovirus type 5 antibody (Abcam, ab6982) was treated in the culture medium at a concentration of 0.1 ng/ml. After 72 hours of virus treatment, the cell killing ability caused by the virus was quantitatively evaluated using a Sigma's cell division kit (XTT assay kit), and in order to visualize the cell killing ability caused by the virus, the 96-well plate was dyed using a crystal violet reagent.

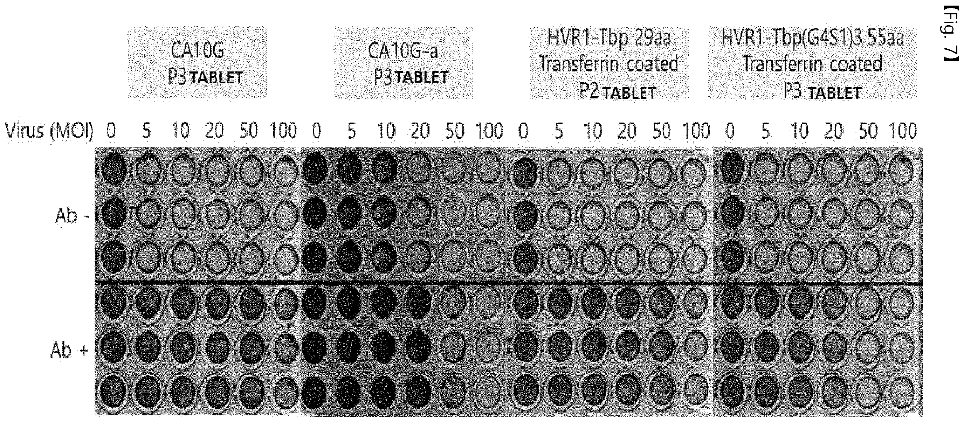

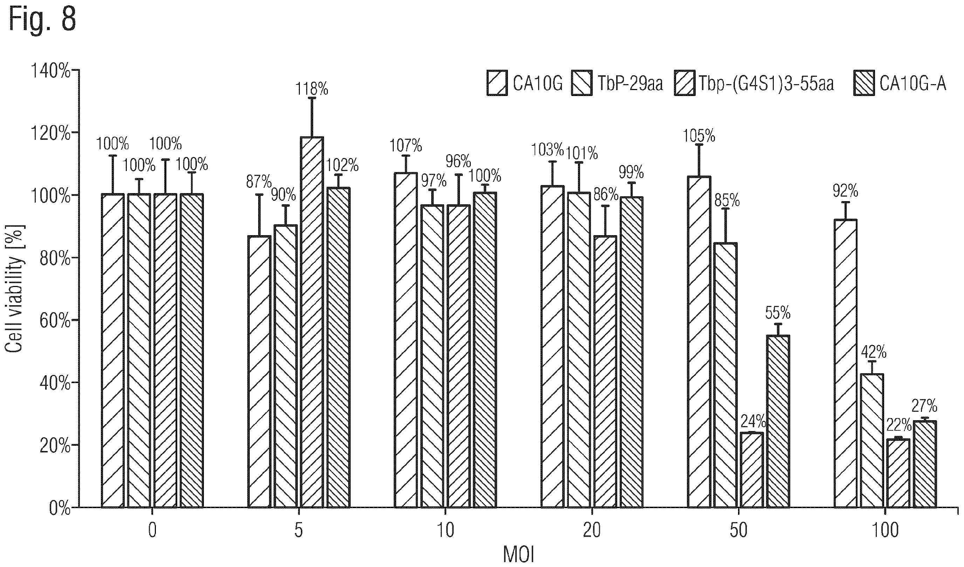

As a result, it was confirmed that a HVR1-Tbp(G₄S₁)₃ 55aa (Tbp-(G₄S₁)₃-55aa) virus had better antibody evasion ability than the positive control CA10G-A at 50 multiplicity of infection (MOI) (FIGS. 7 and 8).

### Example 3. Preparation of virus including transferrin binding domain at HVR2 position

Adenoviruses HVR2-Tbp 29aa and HVR2-(G₄S₁)₃-Tbp 55aa including a transferrin binding moiety were prepared in the same manner of Example 1 by inserting a base sequence (SEQ ID NO: 1) coding Tbp29aa (SEQ ID NO: 2) and a base sequence coding a fusion protein including a linker (SEQ ID NO: 5) at the N-terminus and the C-terminus of Tbp55aa (SEQ ID NO: 4) into the HVR2 region of the hexon of the adenovirus vector, respectively.

### Example 4. Confirmation of neutralizing antibody evasion ability of antitumor virus including transferrin binding domain at HVR2 position

The neutralizing antibody evasion ability of the adenoviruses HVR2-Tbp 29aa and HVR2-(G₄S₁)₃-Tbp 55aa including the transferrin binding moiety in the HVR2 region of the hexon of the adenovirus vector prepared in Example 3 was compared with that of HVR1-(G₄S₁)₃-Tbp 55aa. Specifically, 100 µl of a lung cancer cell line A549 was dispensed into a 96-well plate to be 1 × 10⁴ cells per well. CA10GT (HVR1-(G₄S₁)₃-Tbp 55aa), HVR2-Tbp 29aa (adenovirus type 5/3 including a transferrin binding moiety of SEQ ID NO: 1 in the HVR2 region) and HVR2-(G₄S₁)₃-Tbp 55aa (adenovirus type 5/3 including a transferrin binding moiety of SEQ ID NO: 3 in the HVR2 region and a linker) were ligated at 4°C for 6 hours in a culture medium containing 2 mg/ml of transferrin, treated to the dispensed cells at 0, 5, 10, 20, 50, and 100-fold the cell number, respectively (CA10G: 3.38 × 10¹⁰ ifu/ml; CA10GT: 8.03 × 10⁹ ifu/ml; HVR2-29aa: 6.94 × 10¹⁰ ifu/ml; and HVR2-(G₄S₁)₃-55aa: 1.29 × 10¹⁰ ifu/ml), and then treated with an adenovirus type 5 antibody (Abcam, ab6982) at a concentration of 0.1 ng/ml. After 72 hours of virus treatment, the cell killing ability caused by the virus was quantitatively evaluated using a Sigma's cell division kit (XTT assay kit).

As a result, it was shown that only CA10GT, in which the transferrin binding moiety was inserted into the HVR1 region of the hexon of the adenovirus vector, bound to transferrin in vivo to evade the antibody attack and then kill the cancer cells, and the viruses, in which the transferrin binding moiety was inserted into the HVR2 region of the hexon of the adenovirus vector, showed little antibody evasion ability (FIG. 9).

## Claims

1. An adenovirus comprising a nucleic acid coding a transferrin binding moiety in a coding region of a hypervariable region (HVR) of a hexon protein.

2. The adenovirus of claim 1, wherein the adenovirus comprises a nucleic acid sequence represented by SEQ ID NO: 1, 3, 8 or 10.

3. The adenovirus of claim 1, wherein the transferrin binding moiety includes an amino acid sequence represented by SEQ ID NO: 2 or 4.

4. The adenovirus of claim 1, wherein the N-terminus, the C-terminus, or both the N-terminus and the C-terminus of the transferrin binding moiety is linked to the hexon protein via a linker.

5. The adenovirus of claim 4, wherein the linker includes an amino acid sequence represented by SEQ ID NO: 5.

6. The adenovirus of claim 1, wherein the hypervariable region of the hexon protein is HVR1.

7. The adenovirus of claim 6, wherein the HVR1 of the hexon protein includes an amino acid sequence represented by SEQ ID NO: 7.

8. The adenovirus of claim 1, wherein the nucleic acid coding the transferrin binding moiety is included between codons expressing amino acids at positions 154 and 155 of the base sequence coding the hexon.

9. The adenovirus of claim 1, wherein when the hexon protein is assembled into a capsid of the adenovirus, the transferrin binding moiety is located on the outer surface of the hexon protein.

10. The adenovirus of claim 1, wherein the adenovirus is a human adenovirus.

11. The adenovirus of claim 10, wherein the human adenovirus is selected from the group consisting of human adenovirus serotypes 1 to 57.

12. The adenovirus of claim 10, wherein the human adenovirus is a human adenovirus serotype 5.

13. The adenovirus of claim 10, wherein the human adenovirus is a human adenovirus serotype 5/3.

14. The adenovirus of claim 1, further comprising: a tissue-specific promoter or a tumor-specific promoter.

15. The adenovirus of claim 14, wherein the promoter is operably linked to an endogenous gene of the adenovirus.

16. The adenovirus of claim 14, wherein the promoter is selected from the group consisting of an E2F promoter, a telomerase hTERT promoter, a tyrosinase promoter, a prostate-specific antigen promoter, an alpha-fetoprotein promoter, and a COX-2 promoter.

17. The adenovirus of claim 15, wherein the endogenous gene of the adenovirus has a structure of 5'-ITR-C1-C2-C3-C4-C5 3-TTR;
wherein the C1 includes E1A, E1B or E1A-E1B;
the C2 includes E2B-L1-L2-L3-E2A-L4,
the C3 includes E3 or not;
the C4 includes L5; and
the C5 includes E4 or not.

18. The adenovirus of claim 17, wherein an IRES sequence is further included between E1A and E1B.

19. The adenovirus of claim 17, wherein the promoter is operably linked to E1A and E1B of the endogenous gene of the adenovirus.

20. The adenovirus of claim 1, further comprising: an expression cassette expressing a foreign gene.

21. The adenovirus of claim 20, wherein the expression cassette is included in the E3 region of the endogenous gene of the adenovirus.

22. The adenovirus of claim 1, wherein the adenovirus is an antitumor adenovirus.

23. The adenovirus of claim 1, wherein the adenovirus is an oncolytic adenovirus.

24. The adenovirus of claim 1, further comprising: a capsid modification to increase the infectivity of the adenovirus or to be targeted to a receptor present in a tumor cell.

25. The adenovirus of claim 24, wherein the capsid modification is insertion of an RGD motif within an H1 loop of an adenovirus fiber protein.

26. The adenovirus of claim 24, wherein the capsid modification is substitution of a fiber gene or a part thereof with a homologous part derived from another serotype of adenovirus to form a chimeric adenovirus.

27. The adenovirus of claim 26, wherein the adenovirus comprises a capsid substituted with a fiber gene derived from an adenovirus serotype 3 or a part thereof and a portion excluding the fiber gene includes an adenovirus serotype 5-derived gene.

28. The adenovirus of claim 1, wherein the adenovirus comprises one or more non-adenoviral genes.

29. The adenovirus of claim 28, wherein the non-adenoviral genes are genes used for cancer gene therapy.

30. The adenovirus of claim 29, wherein the genes used for cancer gene therapy are selected from the group consisting of tumor-suppressor genes, genes coding antitumor interfering RNA, and immunostimulatory genes.

31. A pharmaceutical composition for treating cancer comprising the adenovirus of claim 1 as an active ingredient.

32. The pharmaceutical composition for treating cancer of claim 31, wherein the pharmaceutical composition is administered systemically.

33. A use of an adenovirus comprising a nucleic acid coding a transferrin binding moiety in a coding region of a hypervariable region (HVR) of a hexon protein for prevention or treatment of tumors.

34. A method for treating cancer comprising administering an adenovirus comprising a nucleic acid coding a transferrin binding moiety in a coding region of a hypervariable region (HVR) of a hexon protein to a subject suffering from cancer.
